# EUROPEAN PATENT SPECIFICATION

(11) **EP 1 322 350 B1**
(45) Date of publication and mention of the grant of the patent: **30.03.2005**
(21) Application number: 01974313.7
(22) Date of filing: 28.09.2001
(51) Int. Cl.: A61L 31/04, A61L 31/16

(54) **E-PTFE FOIL IMPREGNATED WITH AN ENCAPSULATED BIOACTIVE SUBSTANCE**
MIT VERKAPSELTEN BIOAKTIVEN SUBSTANZEN IMPRÄGNIERTE E-PTFE-FOLIE
FEUILLE DE POLYTETRAFLUOROETHYLENE EXPANSE (E-PTFE) IMPREGNEE D'UNE SUBSTANCE BIOACTIVE ENCAPSULEE

(30) Priority: 28.09.2000 GB 0023807
(43) Date of publication of application: 02.07.2003
(73) Proprietor: Angiomed GmbH & Co. Medizintechnik KG, 76227 Karlsruhe (DE)
(72) Inventor: KLEE, Doris, 52062 Aachen (DE); HILGERS, Christoph, 52249 Eschweiler (DE)
(74) Representative: HOFFMANN - EITLE
(86) International application number: PCT/EP2001/011267
(87) International publication number: WO 2002/026279

(56) References cited:
- US-A- 5 197 882
- US-A- 5 201 778

## Description

This invention relates to an e-PTFE (expanded polytetrafluoroethylene) foil impregnated with a biologically-active substance, to a method of so impregnating a foil and to a prosthesis comprising an e-PTFE impregnated foil, thereby carrying a releasable biologically-active substance.

### Field of the invention

Prostheses, such as stent grafts, can usefully include an e-PTFE membrane, this being inert in the body and capable of providing a useful barrier function. In recent years it has been a challenge to manufacturers of prostheses to invent ways of incorporating biologically active substances in stents, grafts and other prostheses. Growth factors are one such biologically-active substance which would be advantageous to incorporate. Others may include cellular proliferation-controlling or migration-controlling agents, and agents to inhibit thrombosis. The innumerable interstices in an e-PTFE foil (otherwise called herein "membrane" or "film") provide an attractive location for the placement of biologically active substances, but a method has to be found how to load the interstices with the active substance.

The present invention aims to provide one route to achieve such loading.

### Technological Background

US-A-5480711 discloses nano-porous PTFE and its use as a biomaterial.

UA-A-5716660 discloses e-PTFE prostheses impregnated with a solid insoluble biocompatible material, specifically an extracellular matrix protein, such as collagen or gelatin.

US-A-5972027 proposes to load a porous stent with a biomaterial, such as a drug. The drug may be carried in solution and loaded into the pores by imposing a pressure gradient on the solution. The stent is to be made from a metal powder, but the possibility of a stent made from PTFE powder is also mentioned.

EP-A-0706376 discloses use of taxol in the manufacture of a stent. Taxol is disclosed as an anti-angiogenic factor.

US 5 197 882 discloses a periodontal barrier of expanded PTFE containing chemotherapeutic agents encapsulated in microparticles.

### Summary of the invention

It is an object of the present invention to provide e-PTFE foil impregnated with a biologically-active substance.

This object is solved by the subject matter of independent claim 1. Further embodiments are described in dependent claims 2 to 10.

It is another object of the present invention to provide a method of impregnating an e-PTFE foil with a biologically-active substance.

This object is solved by independent claim 11. Further embodiments are described in dependent claims 13 to 18.

It is yet another object of the present invention to provide a prosthesis, such as a stent, comprising an impregnated e-PTFE foil carrying a releasable biologically-active substance.

This object is solved by independent claim 17. A further embodiment is described in dependent claim 18.

In accordance with one aspect of the present invention, there is provided a method of impregnating an e-PTFE foil with a biologically-active substance which involves imposing across the foil a pressure differential sufficient to urge into the interstices of the foil a suspension of nanoparticles in a fluid medium, the nanoparticles containing a desired biologically-active substance. In another aspect, the present invention provides an e-PTFE foil so impregnated. In yet another aspect, the present invention provides a prosthesis comprising an e-PTFE foil so impregnated.

Taking account of typical dimensions of interstices in e-PTFE foils, the range of sizes of nanoparticles which lend themselves to such impregnation will generally lie in a range of from 10 - 1000 nm for average diameters of typically spherical nanoparticles (nanospheres). A preferred range of diameters is from 100 to 800 nm.

Conveniently, the nanoparticles have a surface layer which encapsulates the active substance of interest, and the surface layer is conveniently bioabsorbable and can be of a lactide-containing polymer, such as poly(D,L-lactic-acid), poly(D,L-lactic-acid-co-glycolide) and poly(D,L-lactic-acid-co-trimethylenecarbonate), the latter hereinafter abbreviated to poly(D,L-lactide-co-TMC).

Once the nanoparticles have been urged into the foil interstices, the biologically-active substance delivered by the nanoparticles should be anchored there. One way which the present inventors have found to anchor the nanoparticle load is to perform a heat treatment of the impregnated foil to agglomerate the nanoparticles. Another is to perform a CO₂ procedure in accordance with the CESP process described in *Advanced Engineering Materials 1999,* Vol 1, No. 3-4, pages 206 to 208 in the paper entitled "*Microporous, resorbable implants produced by the CESP process*" by Walter Michaeli and Oliver Pfannschmidt of the "Institut für Kunststoffverarbeitung, Rheinisch-Westfälische Technische Hochschule, D-52062 Aachen, Germany".

For a discussion of preparation techniques and mechanisms of formation of biodegradable nanoparticles from preformed polymers, the reader may refer to a paper by Quintanar-Guerrero, Alleman, Fessi and Doelker which appears in "*Drug Development and Industrial Pharmacy,* 24(12), 1113-1128 (1998)".

In a nutshell, the method preferred herein involves a nanoparticle production step followed by a loading step to impregnate an e-PTFE foil in a pressure cell with a suspension of nanoparticles, followed by an anchoring step to fix in the foil the biologically-active substance carried into the foil by the nanoparticles.

Conveniently, polymeric nanoparticles are manufactured by a solvent evaporation or solvent displacement technique. Loading of the nanoparticles with a medicament or other biologically-active substance is carried out during the nanoparticle manufacturing step.

With more specificity, a base polymer which can be a poly(D,L-lactide), poly(D,L-lactide-co-glycolide) or a poly(D,L-lactide-co-trimethylenecarbonate) is dissolved in a solvent. The solvent can be a water-miscible solvent, such as acetone, when the method is a solvent displacement method.

Alternatively, the solvent can be a water-non-miscible solvent, such as CH₂Cl₂, when the technique is a solvent evaporation technique.

In a second step, the biologically-active substance, such as a drug or medicament, is dissolved in or disbursed in the polymer solution and then the solution is poured into an aqueous phase with continuous stirring. The aqueous phase will likely contain a surfactant or a stabiliser.
Afterwards, the solvent is vacuum-evaporated from the aqueous phase.

The resulting suspension of nanoparticles is transferred into a pressure chamber with continuous stirring. Continuing the stirring, a pressure differential conveniently in the range up to 10 bar is imposed within the pressure cell across a foil workpiece in order that the pressure differential shall drive the suspension into the interstices of the e-PTFE which forms the foil. The procedure is repeated, as many times as is necessary, in order to load the foil workpiece with the specified quantity of nanoparticle material.

For anchoring the nanoparticle material within the interstices of the foil, the impregnated foil can be treated at an elevated temperature to bring about agglomeration or melting of the nanoparticles within the interstices. Alternatively, a treatment with supercritical CO₂ can be utilised. The supercritical CO₂ dissolves the nanoparticles, thereby causing it to flow so that the nanoparticles lose their discrete shape to form a molten structure within the e-PTFE foil. One way of obtaining this molten structure is to follow the procedures advocated by RWTH-Aachen in its CESP process, mentioned above.

It will be appreciated that the loading of the e-PTFE foil with nanoparticles can be accomplished with foil material to be later incorporated into a medical device, or can be incorporated into foil which has already been incorporated in a medical device. The medical device can be a prosthesis, such as a vascular prosthesis. A vascular prosthesis of special interest for the inventors is a vascular stent. The invention will likely find applications for stents which are not vascular stents, such as biliary, ureteral, uretheral, oesophageal, tracheo-bronchial, colorectal, prostatic, hepatic stents.

The pressure differential imposed on the foil in the pressure cell can be achieved by positive pressurisation of the nanoparticle suspension on the upstream side of the foil, or by imposing a sufficiently low enough vacuum on the downstream side of the foil workpiece.

The fluid medium within which the nanoparticles are suspended for the loading step in the pressure cell need not be the same fluid medium in which the nanoparticles are created.

The encapsulation of the biologically-active substance, such as a drug or medication, within the nanoparticles need not be with biodegradable synthetic polymeric materials, such as poly-lactide and its co-polymers, polyesther, polyether, polycyanoacrylate, polyhydroxycarboxylic acid, polyanhydride, polyaminoacids, polyhydroxyalkanoate, but could instead be with bio-compatible, non-biodegradable materials through which, for example, the drug diffuses into the body of the patient, To be considered for this task are, for example, biologically-compatible synthetic polymeric substances. These include silicone, polyalcane, polytetrafluorethylene (PTFE, e-PTFE), polyethylene, such as ultra-pure polyethylene (HOSTALON™ (GUR), LUPULEN™ (UHM)), polypropylene, polyester (such as polyethylene terephthalate or DACRON™, TERYLENE™), polyurethane, as well as polyamides, such as NYLON™, aliphatic and aromatic polyamides (NOMEX, KEVLAR).

### Brief description of the Figures

The present invention will be described, by way of example, with reference to the accompanying figures, of which
- Fig. 1A and 1B: show REM pictures of an untreated and unloaded e-PTFE sample at two magnifications (1A: 2500 fold; 1B: 5000 fold);
- Fig. 2A and 2B: show REM pictures of an e-PTFE layer loaded with nanospheres at two magnifications (2A: 2500 fold; 2B: 5000 fold);
- Fig. 3A and 3B: show REM pictures of an e-PTFE layer after CO₂ treatment (3A: 2500 fold; 3B: 5000 fold);
- Fig. 4A and 4B: show REM pictures of an e-PTFE layer loaded with nanospheres after CO₂ treatment (4A: 2500 fold; 4B: 5000 fold);
- Fig. 5: shows a diagram depicting a release pattern of dexamethasone-loaded nanospheres out of poly-(D,L-lactide-co-TMC) (90:10);
- Fig. 6: shows UV-spectra of dexamethasone-containing PVA-solution;
- Fig. 7: shows a calibration line for dexamethasone in a 4% PVA-solution (M=61000);
- Fig. 8: shows UV-spectra of wash solution contaminated with dexamethasone;
- Fig. 9: shows a calibration line for dexamethasone in water.

### Brief mode/Preferred embodiments

The production of biodegradable spherical nanoparticles by an (o/w)-emulsion-solvent evaporation technique is described below.

### Particle production:

Spherical nanoparticles made of poly(D,L-lactic-acid-co-trimethylenecarbonate) (90:10) were prepared by an (o/w)-emulsion-solvent evaporation technique. 200 mg of the polymer, 40 mg of the model substance dexamethasone in 5 ml of methylenechloride were mixed and cooled down to 0°C. 50 ml of an aqueous 4% (w/v) polyvinylalcohol solution (M=61.000) were added. This mixture was dispersed for 30 s by vortex (Ika Ultra Turrax 25 T basic, Staufen im Breisgau, Germany) at 150.000 rpm at 0°C and then sonicated using an ultrasound generator (Branson Sonifier 450, Danbury, CT, USA) for 120 s to produce the oil in water emulsion. The solution was poured into an 500 ml flask and the organic phase was removed for 30 min under stepwise reduction of the pressure (room pressure down to >60 mbar).

### Particle loading:

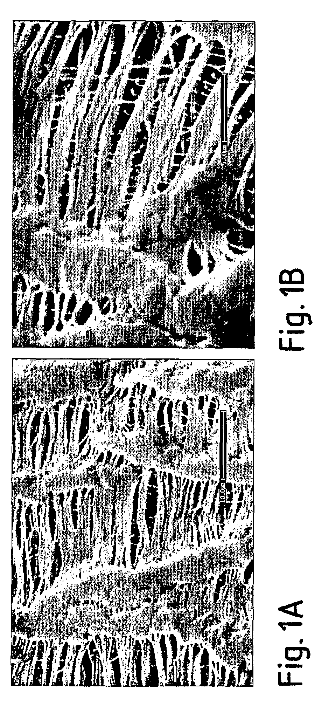

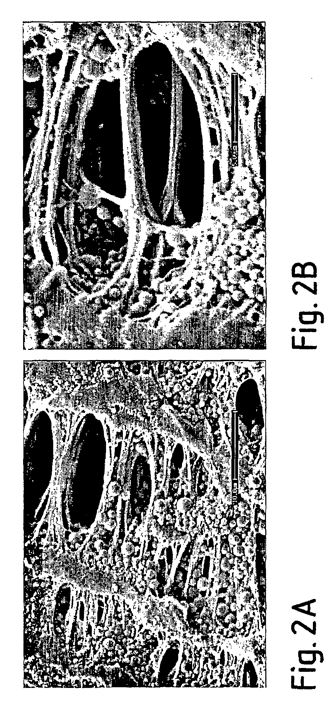

Next, the nanosphere suspension was directly used to incorporate the spheres into the e-PTFE layer by pressing the nanosphere suspension under pressure through an e-PTFE covered stent to obtain a loading of the foil with nanoparticles. The stent was freeze-dried and analysed with REM. Figure 1A and 1B show REM pictures of the untreated and unloaded e-PTFE foil in 2500 fold (Figure 1A) and 5000 fold magnification (Figure 1B). Figure 2A and 2B show the e-PTFE layer loaded with nanospheres at magnification levels of 2500 fold (Figure 2A) and 5000 fold (Figure 2B), respectively.

The particle size distribution of the remaining suspension and the filtrate were measured (refer to Table 1 below) using a particle sizer (Malvern instruments, Mastersizer 2000 with dispersing unit Hydro 2000, Worcestershire, Great Britain). Table 1 shows the computed diameter d of the median particle (n=0.5) of the particle distribution.

**Table 1:**

| d(n=0.5) values of the remaining suspension and filtrate compared with the initial d(n=0.5) value. | | |
|---|---|---|
| **Initial Suspension** **d(n=0.5) in nm** | **Remaining Suspension** **d(n=0.5) in nm** | **Filtrate** **d(n=0.5) in nm** |
| 542 | 991 | 256 |

The remaining suspension shows a higher d(n,0.5) value compared with the freshly produced initial suspension. The amount of small spheres decreases in the suspension during the loading process. The filtrate shows a lower median d(n,0.5) value of 256 nm due to the filter effect of the e-PTFE layer which restrains spheres over approximately 300 nm in diameter.

### Anchoring:

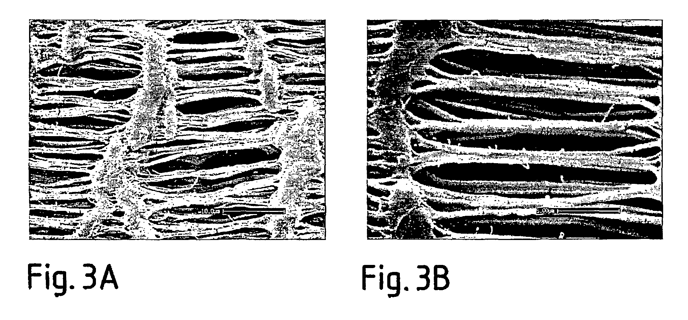

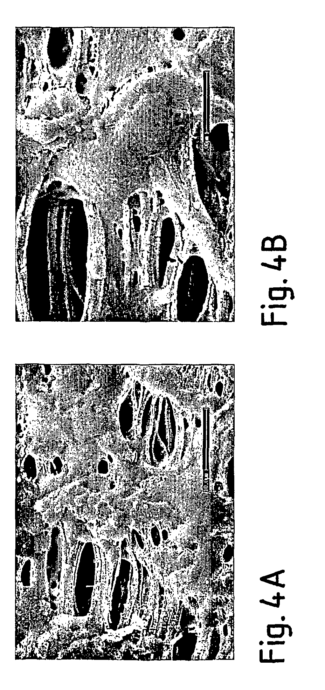

For anchoring the dexamethasone carried by the poly(D,L-lactide-co-TMC) (90:10)-nanospheres in an e-PTFE-matrix, the nanosphere-loaded e-PTFE foil was subsequently treated with supercritical CO₂ for 30 minutes under a pressure of 305 bar. REM pictures were taken, and are presented herein at two different magnification levels of 2500 fold (Figures 3A and 4A) and 5000 fold (Figures 3B and 4B), respectively, in Figures 3A, 3B without nanosphere-loaded e-PTFE and 4A, 4B with nanosphere-loaded e-PTFE. The nanospheres lose their spherical shape due to their solubility in supercritical CO₂.

The remaining e-PTFE matrix is insoluble in supercritical CO₂ and is surrounded by the dissolved poly(D,L-lactide-co-TMC).

Additionally, a release experiment of the hydrophobic model substance dexamethasone from poly(D,L-lactide-co-TMC) (90:10) was performed. Here, the ratio (90:10) refers to the weight ratio of lactide to TMC. Dexamethasone-loaded nanospheres were used for the release experiments. The median d(n=0.5) of the nanospheres was 216 nm.

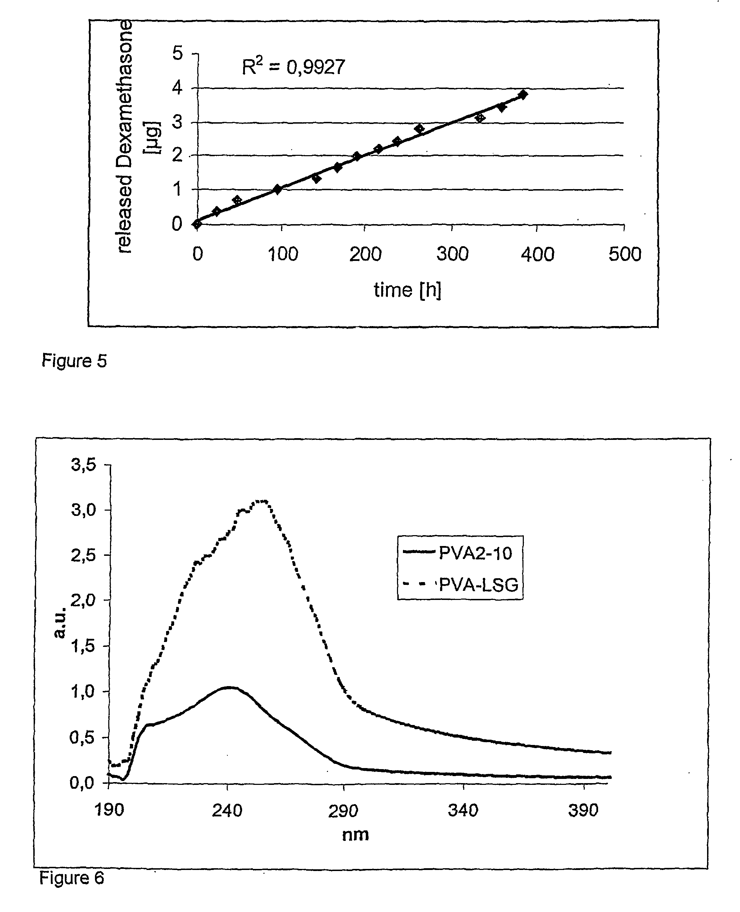

The nanospheres were made by the o/w solvent evaporation technique with a content of 20 % (w/v) dexamethasone with respect to the initial amount of polymer. Figure 5 shows the release pattern. The straight solid line indicates a line of best fit, the result of a least square analysis of the experimental data points. The variance of R²=0.9927 is also shown. The variance being very close to 1.0 and the very good agreement of the experimental data points with the straight line suggest that the rate at which dexamethasone is released from the nanospheres does not change with time. Therefore, a constant amount of dexamethasone migrates through the encapsulation of the nanospheres.

Next, the amount of dexamethasone incorporated into the nanospheres was determined, so that the effective drug loading could be calculated. The loading was determined to be 11.39 %.

During the incorporation of drugs into nanoparticles according to the emulsion solvent evaporation process, a large quantity of the drug migrates into the aqueous PVA-solution. Also during the subsequent solvent washing, migration into the aqueous phase occurs. The content of dexamethasone in the PVA-solution and in the wash-water was determined using UV-spectroscopy. Figure 6 depicts the UV-spectrum of the PVA-solution (solid line) used for the production of the nanospheres containing dexamethasone.

A 4% (w/v) PVA-solution (M=61.000) as a standard was measured. The measurement of the pure PVA-solution yielded pronounced extinctions. For this reason, the solution was diluted in a ratio of 2 to 8 (2 parts of dexamethasone containing PVA-solution to 8 parts of a 4% PVA-solution). Reference is made to Figure 6 (curve PVA-2-10) . The λₘₐₓ-value of dexamethasone was determined to be 242.3 nm.

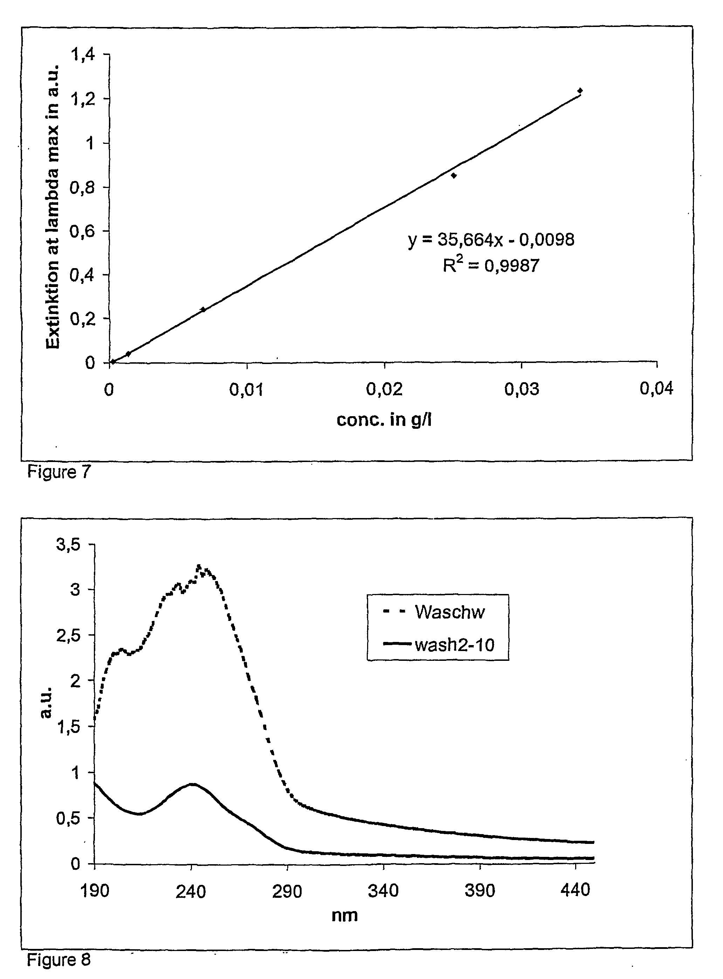

Figure 7 shows the calibration line for dexamethasone in a 4% PVA-solution (M=61.000). The respective extinctions at λₘₐₓ were measured for different dexamethasone concentrations. The straight line indicates a least square analysis line of best fit of the experimental data points. The equation of the linear regression is also shown. Good agreement of the experimental values was obtained with the linear regression fit.

Subsequently, the content of dexamethasone in the wash water solution was measured.

Figure 8 shows the UV-spectra of dexamethasone in the wash-water solution. The samples were measured with respect to water as a standard. The content of dexamethasone in the solution again yielded pronounced extinctions. The sample was diluted in a ratio of 2 to 8 (2 parts of wash water to 8 parts of distilled water).

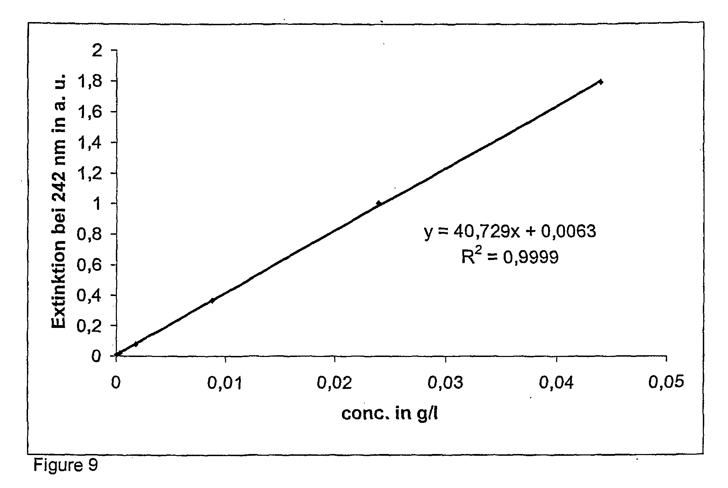

Figure 9 shows the calibration line for dexamethasone in distilled water. The respective extinctions at λₘₐₓ = 242 nm were measured for different dexamethasone concentrations. The straight line indicates a least square fit of the experimental data points. The equation of the linear regression is also shown. Again, good agreement of the experimental values with the linear regression fit was obtained.

The concentration of dexamethasone in the PVA-2-10 sample was determined (c=29.53*10⁻³ g/l). Hence, the amount of dexamethasone in the diluted PVA-2-10 sample (=10 ml) amounts to 2.953*10⁻⁴ g. Consequently, the same amount exists in the 2 ml undiluted PVA-solution. 50 ml of the PVA solution were used during the production process and the amount of dexamethasone can be calculated (refer to Table 2). The amount of dexamethasone in the wash solution was calculated in the same manner.

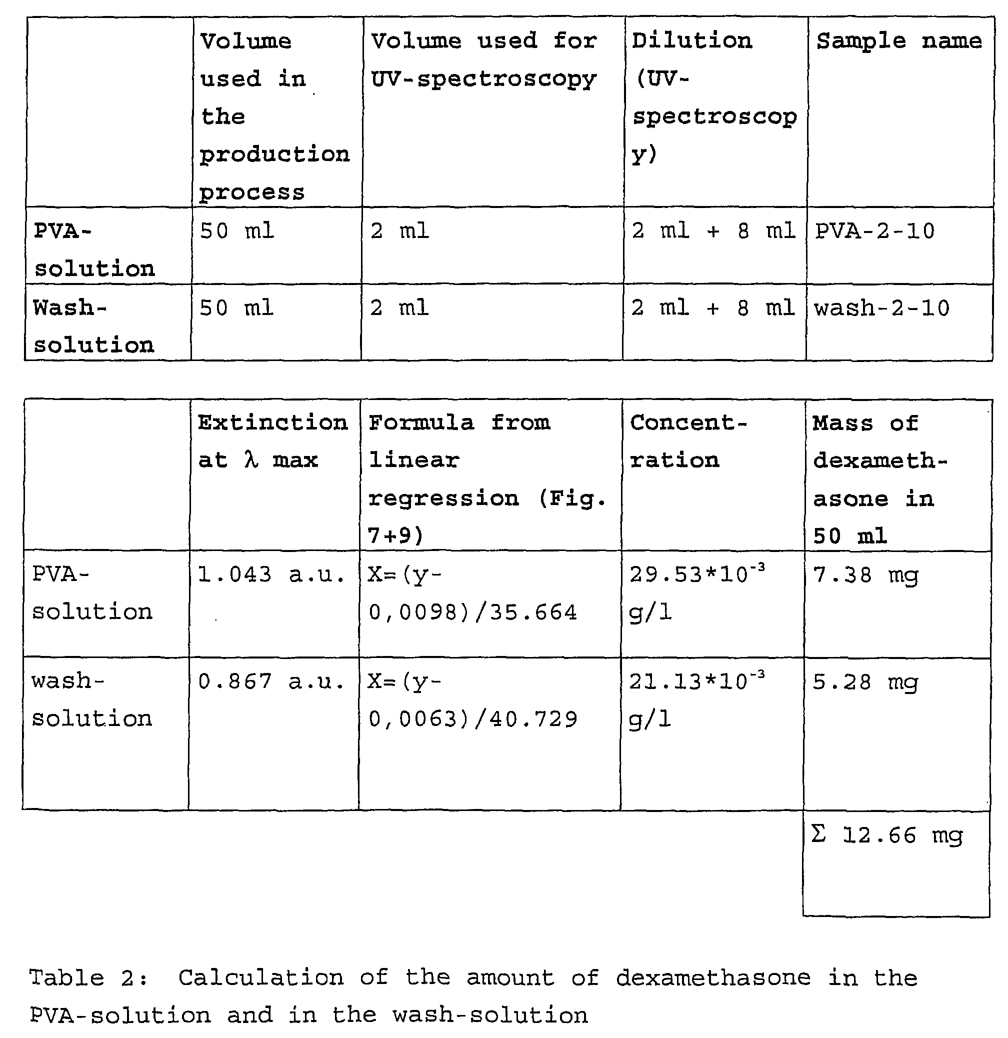

During production, 200 mg of the polymer and 40 mg of dexamethasone were used. 12.66 mg migrated into the aqueous phases, so it can be assumed that 27.34 mg of dexamethasone was incorporated into the spheres corresponding to 11.39 %.

In the foregoing, the following abbreviations have been used:
- o/w:: oil in water
- w/v:: weight per volume
- REM:: Raster Electron Microscopy

The best mode of the invention has been described above only for the model substance dexamethasone. Examples of biologically-active substances to be anchored in an e-PTFE matrix instead of hydrophobic dexamethasone are: hydrophilic substances, such as acryflavine-hydrochloride; rapamycin (sirolimus); taxol (paclitaxel); NO donors or other agents directly or indirectly causing an increase of local NO concentration; growth factors to enhance endothelialisation (e.g. VEGF); growth inhibitors; growth receptor antagonists; antithrombotic agents (e.g. heparin, GPIIb/IIIa receptor antagonists, hirudin); matrix metalloproteinase inhibitors; anti-inflammatory agents; antiproliferative agents (e.g. anti-tumor drugs, e.g. mitomycin C, 5-FU, cisplatin, gemcitabine, radioactive nuclides, adriamycine, mitoxantrone); antisense agents (ogligonucleotides or related compounds blocking transcription/translation process); antimigratory drugs; antioxidants; agents promoting or inhibiting apoptosis; genes for genetransfer (naked or packaged in vectors); hormones, somatostatin analogs; antibodies; angiogenesis inhibitors/promoters.

A supercritical CO₂ has been used for anchoring the biological active substance in the ePTFE foil. However, it is conceivable to use other supercritical agents, such as supercritical nitrogen, which do not harm the biologically active substance.

## Claims

1. e-PTFE foil impregnated with an encapsulated biologically-active substance, **characterised in that** the biologically-active substance is encapsulated in nanoparticles, and that the nanoparticles are agglomerated in the interstices of the e-PTFE foil.

2. Foil as claimed in claim 1, wherein the nanoparticles are generally spherical.

3. Foil as claimed in claim 2, wherein the generally spherical nanoparticles have a diameter which, on average, is in a range of from 10 nm to 1000 nm.

4. Foil as claimed in claim 3, wherein the range of average diameters extends from 100 to 800 nm.

5. Foil as claimed in any one of the preceding claims, wherein the biologically-active substance is encapsulated in a biodegradable substance.

6. Foil as claimed in claim 5, wherein the biodegradable substance is composed of a lactide-containing polymer.

7. Foil as claimed in claim 6, wherein the lactide-containing polymer is poly(D,L-lactic-acid-co-trimethylenecarbonate).

8. Foil as claimed in any one of claims 1 to 4, wherein the biologically-active substance is encapsulated in a material of biologically-compatible, non-biodegradable substance.

9. Foil as claimed in claim 8, wherein the bio-compatible, non-biodegradable substance is a synthetic polymeric substance.

10. Method of impregnating an e-PTFE foil with a biologically-active substance, comprising the steps of:
1. providing the substance encapsulated in nanoparticles by a surface layer of biologically-compatible substance of the nanoparticles;
2. suspending the nanoparticles in a fluid medium;
3. imposing a pressure differential across the foil to urge into the interstices of the foil a flow of said fluid medium which carries the nanoparticles into the interstices; and
4. agglomerating the nanoparticles to anchor in the foil the material of the nanoparticles which have been urged by the pressure differential into the foil interstices.

11. Method according to claim 10, wherein the pressure differential is in a range up to 10 bar.

12. Method as claimed in claim 10 or 11, wherein the agglomerating step is achieved by heating the foil.

13. Method as claimed in claim 10 or 11, wherein the agglomerating step is achieved by dissolving the surface layer of bio-compatible substance to anchor in the foil the material of the nanoparticles which have been urged by the pressure differential into the foil interstices by selecting as the biologically-compatible material a substance which is soluble in a liquid agent, contacting the nanoparticles with the liquid agent, and creating a molten structure so that the material urged into the interstices of the e-PTFE foil is anchored.

14. Method as claimed in claim 13, wherein the liquid agent is supercritical CO₂.

15. Method as claimed in claim 14, and further including the step of treating the e-PTFE foil with supercritical CO₂.

16. Method as claimed in any one of claims 13 to 15, wherein the step of contacting the nanoparticles with the liquid agent runs for 30 minutes at a pressure of 305 bar.

17. Prosthesis comprising an e-PTFE impregnated foil as claimed in any one of claims 1 to 9 and carrying a releasable biologically-active substance.

18. Prosthesis as claimed in claim 17, wherein the prosthesis is a stent comprising a layer of e-PTFE impregnated foil.

## Patentansprüche

1. e-PTFE Folie, die mit einer eingekapselten biologisch aktiven Substanz imprägniert ist, **dadurch gekennzeichnet, dass** die biologisch aktive Substanz in Nanopartikel eingekapselt ist, und dass die Nanopartikel in den Zwischenräumen der E-PTFE Folie agglomeriert sind.

2. Folie nach Anspruch 1, bei der die Nanopartikel im allgemeinen sphärisch sind.

3. Folie nach Anspruch 2, bei der die im allgemeinen sphärischen Nanopartikel einen Durchmesser besitzen, der durchschnittlich in einem Bereich von 10 nm bis 1000 nm liegt.

4. Folie nach Anspruch 3, bei der der durchschnittliche Durchmesser im Bereich von 100 bis 800 nm liegt.

5. Folie nach einem der vorhergehenden Ansprüche, bei der die biologisch aktive Substanz in einer biologisch abbaubaren Substanz eingekapselt ist.

6. Folie nach Anspruch 5, bei der die biologisch abbaubare Substanz aus einem Lactid-haltigen Polymer besteht.

7. Folie nach Anspruch 6, bei der das Lactid-haltige Polymer poly(D,L-Milchsäure-Co-trimethylcarbonat) ist.

8. Folie nach einem der Ansprüche 1 bis 4, bei der die biologisch aktive Substanz in einem Material aus einer biologisch kompatiblen, nicht biologisch abbaubaren Substanz eingekapselt ist.

9. Folie nach Anspruch 8, bei der die biokompatible, nicht biologisch abbaubare Substanz eine synthetische polymerische Substanz ist.

10. Verfahren zum Imprägnieren einer e-PTFE Folie mit einer biologisch aktiven Substanz, mit den Schritten:
1. Vorsehen der in Nanopartikeln eingekapselten Substanz durch eine Oberflächenschicht aus einer biologisch kompatiblen Substanz der Nanopartikel;
2. Suspendieren der Nanopartikel in einem fluiden Medium;
3. Ausüben eines Druckgradienten quer zur Folie, um in die Zwischenräume der Folie einen Fluss des fluiden Mediums zu drängen, das die Nanopartikel in die Zwischenräume einbringt; und
4. Agglomerieren der Nanopartikel, um das Material der Nanopartikel, die durch den Druckgradienten in die Zwischenräume der Folie gedrängt worden sind, in der Folie zu verankern.

11. Verfahren nach Anspruch 10, bei dem der Druckgradient in einem Bereich bis zu 10 bar liegt.

12. Verfahren nach Anspruch 10 oder 11, bei dem der Agglomerationsschritt durch Aufwärmen der Folie erzielt wird.

13. Verfahren nach Anspruch 10 oder 11, bei dem der Agglomerationsschritt durch Auflösen der Oberflächenschicht der biokompatiblen Substanz erzielt wird, um das Material der Nanopartikeln in der Folie zu verankern, die durch den Druckgradienten in die Zwischenräume der Folie gedrängt worden sind, dadurch, dass als das biokompatible Material eine Substanz ausgewählt wird, die in einer Flüssigkeit lösbar ist, und dass die Nanopartikel mit der Flüssigkeit in Kontakt treten, und dass eine geschmolzene Struktur erzeugt wird, so dass das in die Zwischenräume der e-PTFE Folie gedrängte Material verankert wird.

14. Verfahren nach Anspruch 13, bei dem das Flüssigkeitsmittel superkritisches CO₂ ist.

15. Verfahren nach Anspruch 14, des weiteren mit dem Schritt des Behandelns der e-PTFE Folie mit superkritischem CO₂.

16. Verfahren nach einem der Ansprüche 13 bis 15, bei dem der Schritt des Kontaktierens der Nanopartikel mit dem Flüssigkeitsmittel für 30 Minuten bei einem Druck von 305 bar andauert.

17. Prothese, die eine e-PTFE imprägnierte Folie nach einem der Ansprüche 1 bis 9 aufweist und eine freigebbare biologisch aktive Substanz enthält.

18. Prothese nach Anspruch 17, bei der die Prothese ein Stent ist, der eine Schicht aus einer e-PTFE imprägnierten Folie aufweist.

## Revendications

1. Feuille en PTFE-e imprégnée d'une substance biologiquement active encapsulée, **caractérisée en ce que** la substance biologiquement active est encapsulée dans des nanoparticules, et **en ce que** les nanoparticules sont agglomérées dans les interstices de la feuille en PTFE-e.

2. Feuille selon la revendication 1, dans laquelle les nanoparticules sont généralement sphériques.

3. Feuille selon la revendication 2, dans laquelle les nanoparticules généralement sphériques ont un diamètre qui, en moyenne, se situe dans la plage de 10 nm à 1000 nm.

4. Feuille selon la revendication 3, dans laquelle la plage des diamètres moyens s'étend de 100 à 800 nm.

5. Feuille selon l'une quelconque des revendications précédentes, dans laquelle la substance biologiquement active est encapsulée dans une substance biodégradable.

6. Feuille selon la revendication 5, dans laquelle la substance biodégradable est composée d'un polymère contenant du lactide.

7. Feuille selon la revendication 6, dans laquelle le polymère contenant du lactide est le poly(D,L-acide lactique-co-triméthylènecarbonate).

8. Feuille selon l'une quelconque des revendications 1 à 4, dans laquelle la substance biologiquement active est encapsulée dans un matériau d'une substance non biodégradable biologiquement compatible.

9. Feuille selon la revendication 8, dans laquelle la substance non biodégradable biocompatible est une substance polymère synthétique.

10. Procédé d'imprégnation d'une feuille en PTFE-e avec une substance biologiquement active, comprenant les étapes consistant à :
1. obtenir la substance encapsulée dans des nanoparticules à partir d'une couche superficielle de substance biologiquement compatible des nanoparticules ;
2. mettre les nanoparticules en suspension dans un milieu liquide ;
3. exercer une pression différentielle sur la feuille pour pousser dans les interstices de la feuille un flux dudit milieu liquide qui transporte les nanoparticules dans les interstices ; et
4. agglomérer les nanoparticules pour ancrer dans la feuille le matériau des nanoparticules ayant été pousséees par la pression différentielle dans les interstices de la feuille.

11. Procédé selon la revendication 10, dans lequel la pression différentielle se situe dans une plage allant jusqu'à 10 bars.

12. Procédé selon la revendication 10 ou 11, dans lequel l'étape d'agglomération est effectuée en chauffant la feuille.

13. Procédé selon la revendication 10 ou 11, dans lequel l'étape d'agglomération est effectuée en dissolvant la couche superficielle de substance biocompatible pour ancrer dans la feuille le matériau des nanoparticules ayant été poussées par la pression différentielle dans les interstices de la feuille en choisissant comme matériau biologiquement compatible une substance qui est soluble dans un agent liquide, en mettant les nanoparticules en contact avec l'agent liquide, puis en créant une structure fondue, de façon à ce que le matériau poussé dans les interstices de la feuille en PTFE-e soit ancré.

14. Procédé selon la revendication 13, dans lequel l'agent liquide est du CO₂ supercritique.

15. Procédé selon la revendication 14, et comprenant en outre l'étape consistant à traiter la feuille en PTFE-e avec du CO₂ supercritique.

16. Procédé selon l'une quelconque des revendications 13 à 15, dans lequel l'étape consistant à mettre les nanoparticules en contact avec l'agent liquide dure 30 minutes à une pression de 305 bars.

17. Prothèse comprenant une feuille en PTFE-e imprégnée selon l'une quelconque des revendications 1 à 9 et transportant une substance biologiquement active libérable.

18. Prothèse selon la revendication 17, laquelle prothèse est une endoprothèse comprenant une couche de feuille en PTFE-e imprégnée.
